# EUROPÄISCHE PATENTSCHRIFT

(11) **EP 3 032 209 B1**
(45) Veröffentlichungstag und Bekanntmachung des Hinweises auf die Patenterteilung: **06.09.2017**
(21) Anmeldenummer: 15003372.8
(22) Anmeldetag: 25.11.2015
(51) Int. Cl.: F28F 9/02, F28D 7/00, F28D 7/10, F28D 7/16, F28F 13/08, F28D 21/00

(54) **QUENCHKÜHLSYSTEM**
QUENCH COOLING SYSTEM
SYSTEME DE REFROIDISSEUR A INJECTION

(30) Priorität: 11.12.2014 DE 102014018261
(43) Veröffentlichungstag der Anmeldung: 15.06.2016
(73) Patentinhaber: Borsig GmbH, 13507 Berlin (DE)
(72) Erfinder: Carsten, Birk, 16548 Glienicke (DE)
(74) Vertreter: Radünz, Ingo

(56) Entgegenhaltungen:
- EP-A1- 0 070 371
- EP-A1- 0 290 812
- DE-A1- 4 445 687
- DE-U1- 7 827 519

## Beschreibung

Die Erfindung betrifft ein Quenchkühlsystem mit den Merkmalen des Oberbegriffs des Patentanspruchs 1.

Bei einigen Anlagen zur Äthylenherstellung werden Spaltgasöfen oder Cracking Furnaces in einem zweistufigen Kühlsystem verwendet. Dabei wird gewöhnlich ein vertikal angeordneter Doppelrohrwärmetauscher als primärer Quenchkühler und ein herkömmlicher vertikal oder horizontal angeordneter Rohrbündel-Wärmetauscher als sekundärer Quenchkühler vorgesehen.

Ein solcher Rohrbündel-Wärmetauscher dient als Prozessgas-Abhitzekessel zur schnellen Abkühlung von Reaktionsgasen aus Spaltgasöfen oder Chemieanlagen-Reaktoren bei gleichzeitiger Erzeugung von Hochdruckdampf als die entstehende Wärme abführendes Kühlmedium.

Aus der EP 0 417 428 B1 ist ein Rohrbündel-Wärmetauscher bekannt, bei dem zumindest ein Rohrbündel von einem Mantel unter Bildung eines Innenraumes umschlossen ist, der zwischen zwei mit Abstand voneinander angeordneten Rohrböden ausgebildet ist, wobei in den Rohrböden jeweils Rohre der Rohrbündel beidseitig gehalten sind. Dabei ist der Rohrboden auf der Gaseintrittsseite mit die Rohre konzentrisch umgebenden offenen Ausdrehungen und parallelen Kühlkanälen versehen, die miteinander in Verbindung stehen und von einem Kühlmedium durchströmt sind.

Aus der WO 01/48434 A1 ist weiter ein Rohrbündel-Wärmetauscher mit unter Druck stehendem Mantel und einer unteren Rohrplatte bekannt, die den Innenraum des Mantels von einem Einlass-Verteilerstück für den Eintritt des zu kühlenden Fluids trennt. Die untere Rohrplatte weist Durchgänge für das Fluid auf, und nahe der inneren Oberfläche der Rohrplatte sind seitlich Reinigungsdurchgänge zur Verbindung mit dem Äußeren des Mantels angeordnet und dafür bestimmt, durch den Mantel eine Vorrichtung einzuführen, um die Rohrplatte am Fuß des Rohrbündels zu reinigen. Es können auch Inspektionsdurchgänge nahe der Plattenoberfläche vorhanden sein, um die zu reinigende Zone visuell zu prüfen.

DE 44 45 687 A1 beschreibt ein Quenchkühlsystem gemäß dem Oberbegriff des Anspruchs 1.

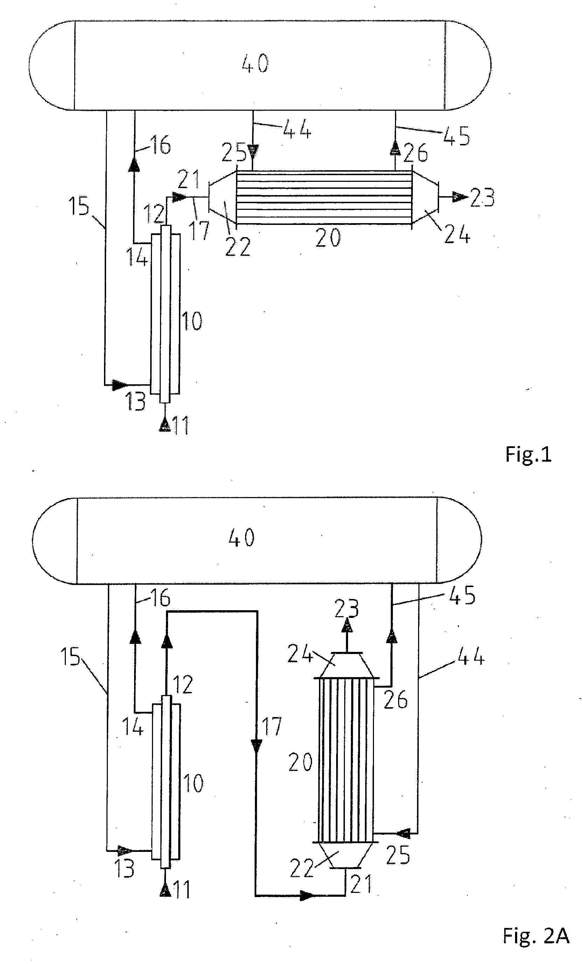

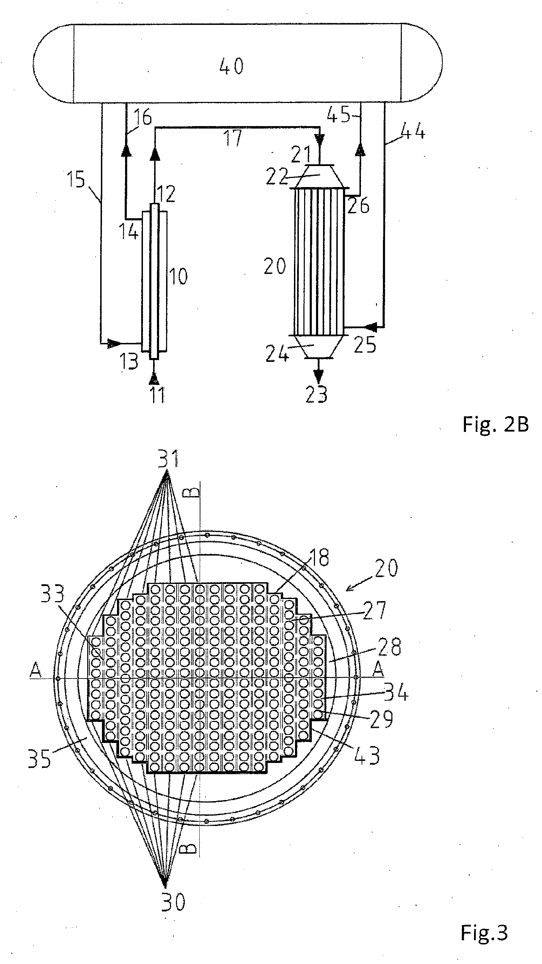

Typische Anordnungen solcher Quenchkühlsysteme sind in Fig. 1 und Fig. 2 schematisch dargestellt. Bei den gezeigten Darstellungen ist der primäre Quenchkühler als Doppelrohrwärmetauscher stets in senkrechter Lage ausgeführt, während der als sekundärer Quenchkühler dienende Rohrbündel-Wärmetauscher einmal in horizontaler Lage gemäß Fig. 1 und das andere Mal in vertikaler Lage in zwei unterschiedlichen Schaltungen für den Gaseintritt und den Gasaustritt nach Fig. 2A und 2B angeordnet ist.
Die Anordnungen der beiden unterschiedlichen primären und sekundären Quenchkühler, die einer gemeinsamen, erhöht angeordneten Dampftrommel dienen, sind die bevorzugten Ausführungen in Verbindung mit der Brennkammer eines Spaltgasofens. In den meisten Fällen werden die Quenchkühler oberhalb des Strahlungsteils des Spaltgasofens angeordnet.
Bei vertikal angeordneten sekundären Quenchkühlern, bei denen der Rohrboden beim Gaseintritt nach Fig.2A oder beim Gasaustritt nach Fig.2B den tiefsten Punkt im Wassersystem darstellt, ist eine hohe Geschwindigkeit des Wasserflusses über den Rohrboden sehr entscheidend, um schädliche Einflüsse in Bezug auf den Rohrboden zu vermeiden. Solche Einflüsse entstehen z. B. durch Ablagerungen infolge von Korrosion und durch Überhitzung infolge von einem Absetzen von festen Partikeln auf dem Rohrboden.
Kleine feste Partikel treten sehr oft in das Wasser des Wasserkreislaufes des Quenchkühlers ein, insbesondere während der Inbetriebnahme einer solchen Anlage, wie zum Beispiel für die Äthylenherstellung. Zusätzlich erzeugen die wasserseitigen Metalloberflächen des Rohrbodens, der Rohre und des Mantels eine Schicht aus Magnetit oder Fe₃O₄. Die Schicht aus Magnetit schützt den Stahl des Rohrbodens und erneuert sich stetig langsam selbst aus der Metalloberfläche bei Betriebstemperatur, wobei eine kleine Menge von Partikeln bestehend aus Magnetit in das Wasser freigesetzt wird.

Neben der hohen Geschwindigkeit des Wasserflusses ist ebenso wichtig, den Wasserfluss über den Rohrboden weg von empfindlichen Bereichen des Rohrbodens, z. B. die Rohrbodenmitte mit dem höchsten Wärmefluss, zu Stellen zu führen, an denen ein wirksames Umwehen oder Blow-Down angewendet werden kann.

Das Rohrbodendesign des sekundären Quenchkühlers ist als sogenanntes Membrandesign ausgebildet und besteht aus einer dünnen Platte mit einer Stärke von ungefähr 25 mm. Die Bündelrohre des Quenchkühlers werden auf die dünne Platte geschweißt.

Es sind keine Vorrichtungen auf der Platte vorgesehen, um den Wasserfluss über den Rohrboden des Gaseintritts oder des Gasaustritts zu führen.

Eine Aufgabe der vorliegenden Erfindung besteht darin, ein Quenchkühlsystem mit einer Mediumflussanordnung zu schaffen, bei welcher der Mediumfluss je nach Schaltung des sekundären Quenchkühlers über den Rohrboden der Gaseintrittsseite oder der Gasaustrittsseite so geführt ist, dass Ablagerungen verhindert werden. Eine weitere Aufgabe besteht darin, zu der Mediumflussanordnung auf dem Rohrboden auf Seiten des Gaseintritts oder des Gasaustritts einen Zugriff vorzusehen, durch den der Rohrboden inspiziert und abhängig von der Inspektion auf einfache Weise gereinigt werden kann.

Die zugrundeliegende Aufgabe wird gelöst durch ein Quenchkühlsystem mit einem primären Quenchkühler als Doppelrohrwärmetauscher und einem Rohrbündelwärmetauscher als sekundären Quenchkühler mit mindestens einem Rohrbündel, wobei das Rohrbündel von einem Mantel unter Bildung eines Mantelraumes umschlossen ist, der zwischen zwei mit Abstand voneinander angeordneten Rohrböden ausgebildet ist, zwischen denen Bündelrohre des Rohrbündels beidseitig in den Rohrböden gehalten sind. Dabei ist der Rohrboden auf der Seite vom Gaseintritt oder Gasaustritt mit den Bündelrohren als dünner Rohrboden in Membrandesign ausgebildet. Der dünne Rohrboden ist mit parallelen Kühlkanälen versehen, die miteinander in Verbindung stehen und von einem Kühlmedium durchströmt sind. Die Kühlkanäle sind in Tunnelanordnung ausgebildet und auf dem dünnen Rohrboden als Rohrplatte angeordnet. Die Kühlkanäle in Tunnelanordnung weisen eine viereckige Tunnelgeometrie auf. Die Kühlkanäle mit Tunnelgeometrie sind gebildet aus dem dünnen Rohrboden, der eine Gasseite von einer Wasser-/Dampfseite trennt und mit einem Ringflansch verbunden ist, der mit dem Mantel des umschlossenen Rohrbündels verbunden ist; aus auf dem Rohrboden angeordneten parallelen Stegen, die mit dem Rohrboden verbunden sind und einzelne Wasser-/Dampfströme voneinander trennen; aus einem mit Öffnungen für Bündelrohre versehenen Abdeckblech, das mit den Stegen verbunden ist und die Strömung in der Tunnelanordnung der Kühlkanäle begrenzt und ein Entweichen der Strömung in einen vom Mantel des eingeschlossenen Rohrbündels umschlossenen Mantelraum bis auf einen vorbestimmten Anteil verschließt. Die in Tunnelanordnung ausgebildeten Kühlkanäle bewerkstelligen eine eindeutig gerichtete Strömung von den Eintrittsöffnungen in Richtung der Austrittsöffnungen der Kühlkanäle.

Als besonders vorteilhaft hat sich herausgestellt, wenn mindestens zwei der jeweiligen Kühlkanäle in Tunnelanordnung eine Veränderung des Querschnitts der Kühlkanäle oder der Tunnel durch eine kontinuierliche Reduzierung der Tunnelhöhe von der Eintrittsöffnung zur Austrittsöffnung um einen vorbestimmten Winkel α aufweisen, der zwischen der senkrechten Linie der Austrittsöffnung und dem Abdeckblech gebildet ist.

Weiterhin hat sich als vorteilhaft erwiesen, wenn der zwischen der senkrechten Linie der Austrittsöffnung eines Kühlkanals und dem Abdeckblech gebildete vorbestimmte Winkel α im Bereich von größer/gleich 90° bis 110° liegt, da der Winkel abhängig ist von der vorbestimmten Erhöhung der erforderlichen Geschwindigkeit der Strömung über vorbestimmte Bereiche des zu kühlenden Rohrbodens.

Als weiteren Vorteil bei einer anderen Ausbildung des erfindungsgemäßen Quenchkühlsystems hat zu gelten, dass auf Höhe der Kühlkanäle in Tunnelanordnung an der äußeren Oberflächenseite des mit dem Mantel verbundenen Ringflansches jeweils gegenüberliegend und fluchtend Inspektions- oder Reinigungsstutzen angebracht sind und dass die Inspektions- oder Reinigungsstutzen über Bohrungen im Ringflansch mit den Kühlkanälen in Tunnelanordnung kommunizieren.

Weiter ist als vorteilhaft festgestellt worden, wenn die den Kühlkanälen zugeordneten und jeweils gegenüberliegend und fluchtend am Ringflansch angebrachten Inspektions- oder Reinigungsstutzen mit Kappen ausgerüstet sind und wenn die Kappen oder einzelne Kappen der jeweils gegenüberliegenden Inspektions- oder Reinigungsstutzen als Öffnung zur wasserseitigen Wartung oder Inspektion der Bündelrohre im Bereich der Kühlkanäle in Tunnelanordnung entfernbar angebracht sind.

Bei dem erfindungsgemäßen Quenchkühlsystem hat sich weiter als vorteilhaft erwiesen, dass die Kappen oder einzelne Kappen der jeweils gegenüberliegenden Inspektions- oder Reinigungsstutzen als Öffnung zur Reinigung von vorhandenen Ablagerungen im Bereich der Kühlkanäle in Tunnelanordnung mit einem Wasserstrahl entfernbar angebracht sind.

Bei einer weiteren Ausbildung des erfindungsgemäßen Quenchkühlsystems hat sich als vorteilhaft herausgestellt, dass die den Kühlkanälen zugeordneten und jeweils gegenüberliegend und fluchtend am Ringflansch angebrachten Inspektions- oder Reinigungsstutzen über die Bohrungen im Ringflansch und über als Weiterführung der Bohrungen an dem Ringflansch angeschweißte Ablaufrohre mit einem an einer Seite auf Höhe der Kühlkanäle in Tunnelanordnung angebrachten Abschlämmsammler kommunizieren.

Weiter ist besonders vorteilhaft, dass die Inspektions- oder Reinigungsstutzen an der den Ablaufrohren gegenüberliegenden Außenseite des Abschlämmsammlers angeordnet sind.

Bei der Ausbildung des Quenchkühlsystems mit dem Abschlämmsammler ist vorteilhaft, dass die Inspektions- öder Reinigungsstutzen gegenüber der Seite mit der Anordnung des Abschlämmsammlers direkt am Ringflansch angeordnet sind.

Das bevorzugte Tunnelflussdesign sichert eine hohe Mediumflussgeschwindigkeit über den Rohrboden der Gaseintrittsseite oder der Gasaustrittsseite. Wegen der hohen Geschwindigkeit des Mediumflusses können sich grundsätzlich nicht irgendwelche festen Partikel auf dem Rohrboden absetzen. Da ein Absetzen von festen Partikeln auf dem Rohrboden im wesentlichen nicht eintritt, können eine Überhitzung des Rohrbodens und eine Heißwasserkorrosion nicht eintreten.

Die Tunnelflussanordnung weist zwei entscheidende Merkmale auf. Erstens setzen sich feste Partikel aufgrund der erzeugten hohen Mediumflussgeschwindigkeit durch die vorteilhafte Tunnelflussanordnung im wesentlichen nicht ab, und zweitens tritt eine Überhitzung des Rohrbodens durch eine vorgesehene geführte intensive Kühlung und somit eine Heißwasserkorrosion nicht ein. Die Tunnelflussanordnung stellt einen kontinuierlichen und gleichförmigen Wasserfluss zu und entlang dem Rohrboden der Gaseintrittsseite oder der Gasaustrittsseite eines vertikal angeordneten sekundären Quenchkühlers sicher, wobei im wesentlichen eine Ablagerung von festen Partikeln und Schlamm auf der Wasserseite verhindert wird.

Auf eine solche Weise wird die Laufzeit und Zuverlässigkeit eines Quenchkühlsystems durch die Ausbildung der vorteilhaften Tunnelflussanordnung auf dem jeweiligen Rohrboden beträchtlich gesteigert.

In weiterer Ausbildung des erfindungsgemäßen Quenchkühlsystems ist in vorteilhafter Weise vorgesehen, dass über die Inspektions- und Reinigungsstutzen bei entfernten Kappen ein durchgehender Zugriff auf jeden auf dem Rohrboden angeordneten Kühlkanal zu erreichen ist, der dann durch Einleiten von Wasser als bevorzugtes Medium unter Hochdruck entweder von beiden Seiten oder von nur einer Seite zu reinigen ist. Das Abschlämmwasser verlässt den Kühlkanal jeweils auf der gegenüberliegenden Seite, bevorzugter Weise über Ablaufrohre in den angebrachten Abschlämmsammler.

Weitere Vorteile der Erfindung sind in der Zeichnung anhand von Ausführungsbeispielen dargestellt und nachfolgend näher beschrieben. Es zeigen:
Fig. 1 ein Quenchkühlsystem mit einem primären Quenchkühler in vertikaler Anordnung und einem sekundären Quenchkühler in horizontaler Anordnung gemäß dem Stand der Technik;
Fig. 2A eine ähnliche Anordnung wie in Fig. 1 mit einem primären Quenchkühler in vertikaler Anordnung und einem sekundären Quenchkühler in einer vertikalen Aufstellung mit einem am unteren Ende angebrachten Gaseintritt gemäß dem Stand der Technik;
Fig. 2B eine ähnliche Anordnung wie in Fig. 2A mit einem am oberen Ende angeordneten Gaseintritt beim sekundären Quenchkühler in einer vertikalen Aufstellung gemäß dem Stand der Technik;
Fig. 3 ein erfindungsgemäßes Quenchkühlsystem mit einer Ausführung von Kühlkanälen in Tunnelanordnung auf einem dünnen Rohrboden eines sekundären Quenchkühlers im Schnitt kurz über dem Rohrboden in verkleinertem Maßstab in Draufsicht;
Fig. 4A ein erfindungsgemäßes Quenchkühlsystem mit einer Ausführung von Kühlkanälen in Tunnelanordnung nach Fig. 3 im Schnitt entlang der Linie A - A;
Fig. 4B ein erfindungsgemäßes Quenchkühlsystem mit einer Ausführung eines Kühlkanals in Tunnelanordnung nach Fig.3 im Schnitt entlang der Linie B - B;
Fig. 5 eine Einzelheit X nach Fig. 4A im vergrößerten Maßstab;
Fig. 6 eine Ausbildung eines Kühlkanals oder Tunnels zur Erhöhung der Mediumflussgeschwindigkeit über einen Rohrboden eines sekundären Quenchkühlers für das erfindungsgemäße Quenchkühlsystem gemäß Fig.4B mit Kühlwassereintritt;
Fig. 7 eine Ausführung für einen Zugang zu angeordneten Kühlkanälen in Tunnelanordnung auf einem dünnen Rohrboden. eines sekundären Quenchkühlers des erfindungsgemäßen Quenchkühlsystems im Schnitt gemäß Fig. 3;
Fig 8 eine Einzelheit Y nach Fig. 7 in einem vergrößerten Maßstab und
Fig. 9 eine Einzelheit Z nach Fig. 7 in einem vergrößerten Maßstab.

Das in Fig. 1 dargestellte Quenchkühlsystem besteht allgemein aus einem vertikal angeordneten Doppelrohrwärmetauscher als primärer Quenchkühler 10 und einem herkömmlichen horizontal angeordneten Rohrbündelwärmetauscher als ein sekundärer Quenchkühler 20. Die Anordnung der beiden unterschiedlichen Quenchkühler, die einer gemeinsamen, erhöht angeordneten Dampftrommel 40 dienen, ist eine der bevorzugten Schaltungen in Verbindung mit der nicht dargestellten Brennkammer eines ebenfalls nicht gezeigten Spaltgasofens nach dem Stand der Technik.

Ein Gaseinlass 11 für einen Gasstrom gemäß Pfeilrichtung ist am unteren Ende des vertikal aufgestellten primären Quenchkühlers 10 angeordnet. Der Gasstrom verlässt am oberen Ende beim Gasauslass 12 den vertikal aufgestellten primären Quenchkühler 10 in einem vorbestimmten abgekühlten Zustand. Der abgekühlte Gasstrom wird über eine zwischen dem Gasauslass 12 des primären Quenchkühlers 10 und einem Gaseintritt 21 eines Eintrittskopfes 22 des horizontal angeordneten sekundären Quenchkühlers 20 angebrachte Rohrleitung 17 dem sekundären Quenchkühler 20 auf der Seite des Gaseintritts zugeführt, um weiter heruntergekühlt zu werden, und verlässt den sekundären Quenchkühler 20 auf der gegenüberliegenden Seite beim Gasaustritt 23 eines Austrittskopfes 24.

Das Kühlmedium, insbesondere Wasser, wird dem primären Quenchkühler 10 aus der Dampftrommel 40 gemäß Pfeilrichtung über eine Zulaufrohrleitung 15 oberhalb des Gaseinlasses 11 beim Kühlwassereinlass 13 zugeführt und verlässt als Wasser-/Dampfgemisch den Quenchkühler über eine Abführrohrleitung 16 unterhalb des Gasauslasses 12 beim Kühlwasserauslass 14 zurück in die Dampftrommel 40. Dem horizontal angeordneten sekundären Quenchkühler 20 wird das Kühlmedium gemäß Pfeilrichtung über eine sekundäre Zulaufrohrleitung 44 hinter dem Eintrittskopf 22 beim Kühlwassereintritt 25 aus der Dampftrommel 40 zugeführt und verlässt als Wasser-/ Dampfgemisch den Quenchkühler vor dem Austrittskopf 24 über einen Kühlwasseraustritt 26 und eine sekundäre Abführrohrleitung 45 zurück zur Dampftrommel.

Solche Quenchkühlsysteme dienen zum schnellen Kühlen von Reaktionsgas oder Spaltgas aus einem Spaltgasofen oder einem Chemieanlagen-Reaktor mit Hilfe von einem unter Hochdruck stehenden, siedenden und teilweise verdampfenden Medium, insbesondere Wasser.

In Fig. 2A ist eine Anordnung eines Quenchkühlsystems gezeigt, bei dem der primäre Quenchkühler 10 und der sekundäre Quenchkühler 20 vertikal unterhalb der Dampftrommel 40 angeordnet sind. Die in Fig. 1 verwendeten Bezugszeichen für die gleichen dargestellten Bauteile bleiben dieselben, so dass eine weitere Beschreibung der schematischen Schaltung im Prinzip entfallen kann. Beim vertikal aufgestellten sekundären Quenchkühler 20 erfolgt die Gaseinspeisung entsprechend Pfeilrichtung wie beim primären Quenchkühler 10 vom unteren Ende des sekundären Quenchkühlers über den Gaseintritt 21 am Eintrittskopf 22. Der Gaseintritt 21 ist über die Rohrleitung 17 mit dem Gasauslass 12 des primären Quenchkühlers 10 verbunden. Das Gas verlässt den vertikal aufgestellten Quenchkühler 20 am oberen Ende des Austrittskopfes 24 beim Gasaustritt 23.

Das Kühlmedium, insbesondere Wasser, aus der Dampftrommel 40 wird in Fig. 2A gemäß Pfeilrichtung über die sekundäre Zulaufrohrleitung 44 beim Kühlwassereintritt 25 oberhalb des Eintrittskopfes 22 dem sekundären Quenchkühler 20 zugeführt und verlässt über die sekundäre Abführrohrleitung 45 beim Kühlwasseraustritt 26 unterhalb des Austrittskopfes 24 den sekundären Quenchkühler 20 zurück in die Dampftrommel 40.

In Fig. 2B ist eine schematische Schaltung ähnlich der in Fig.2A des Quenchkühlsystems gezeigt. Bei der gezeigten Ausführung eines Quenchkühlsystems wird das Gas gemäß Pfeilrichtung über die Rohrleitung 17 vom Gasauslass 12 des vertikal aufgestellten primären Quenchkühlers 10 über den am oberen Ende des vertikal aufgestellten sekundären Quenchkühlers 20 angeordneten Gaseintritt 21 des Eintrittskopfes 22 eingespeist. Das Gas verlässt den vertikal aufgestellten Quenchkühler 20 beim Gasaustritt 23 am unteren Ende des Gasaustrittskopfes 24.

Bei der in Fig. 2B dargestellten Schaltung wird das Kühlwasser aus der Dampf trommel 40 gemäß Pfeilrichtung über die sekundäre Zuführrohrleitung 44 beim Kühlwassereintritt 25 vom unteren Ende oberhalb des Austrittskopfes 24 des vertikal aufgestellten sekundären Quenchkühlers 20 zugeführt und verlässt als Wasser-/Dampfgemisch den Quenchkühler unterhalb des Eintrittskopfes 22 über den Kühlwasseraustritt 26 und die sekundäre Abführrohrleitung 45 zurück in die Dampftrommel 40.

In Fig. 3 ist die Ausbildung von Kühlkanälen 27 in Tunnelanordnung auf einem dünnen Rohrboden 28 des sekundären Quenchkühlers 20 im Schnitt kurz über dem Rohrboden gezeigt. Auf dem dünnen Rohrboden 28 sind die Kühlkanäle 27 als Tunnel parallel angeordnet. Die Kühlkanäle 27 sind auf der durch ein Abdeckblech 34 gebildeten Oberfläche senkrecht zum Rohrboden 28 mit Öffnungen 18 versehen, die mit einem vorbestimmten Abstand angeordnet sind.

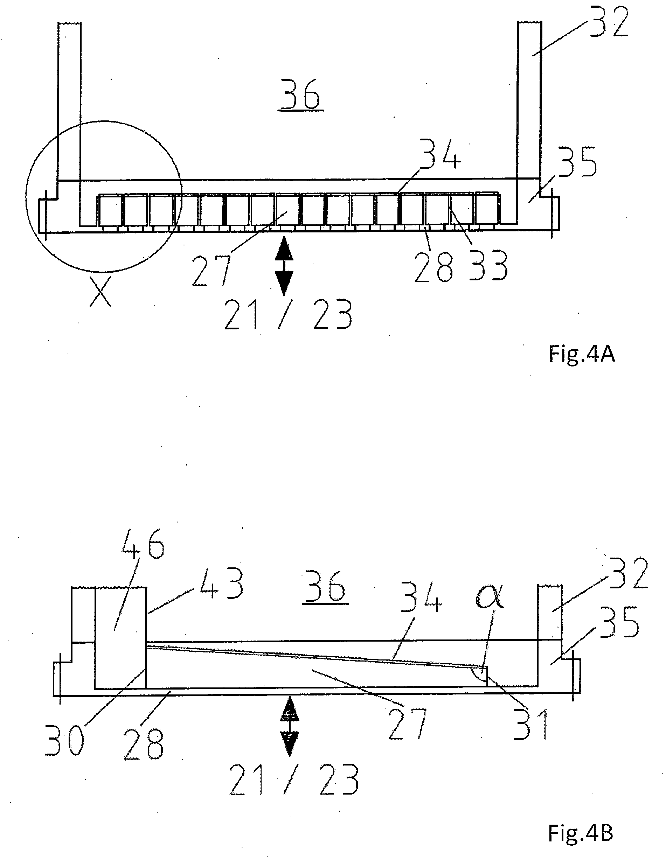

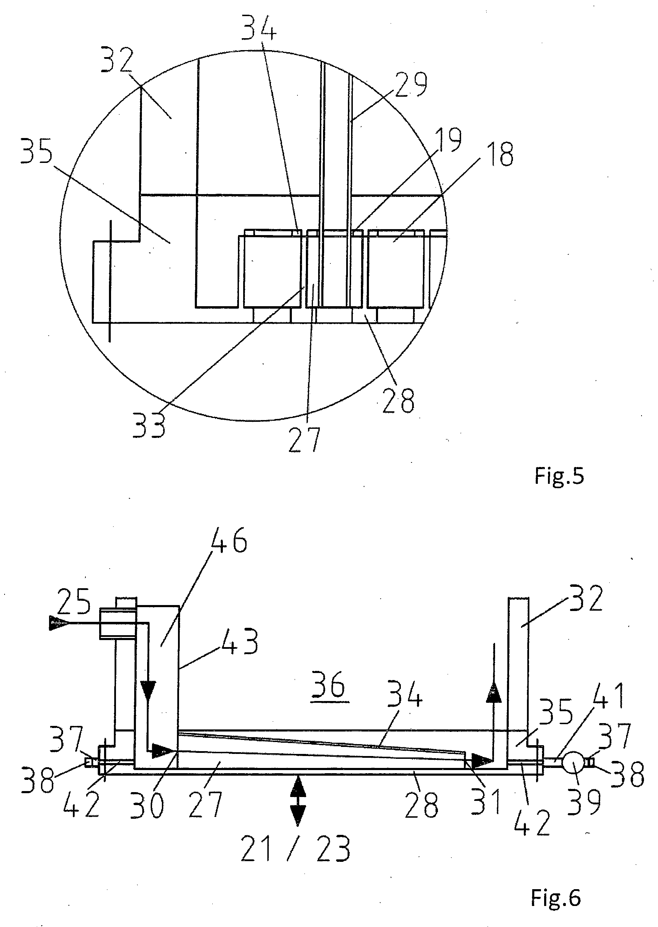

Wie aus Fig. 3 in Verbindung mit Fig. 4, 4B und Fig. 5 besser zu entnehmen ist, sind durch die Öffnungen 18 senkrecht zum Rohrboden 28 mit Abstand voneinander Bündelrohre 29 von Rohrbündeln mit einem zwischen Öffnung und Bündelrohr ausgebildeten Ringspalt 19 hindurchgeführt, der zwischen der jeweiligen Öffnung 18 und dem durchgeführten Bündelrohr 29 vorbestimmt ist. Die einen Enden der Bündelrohre 29 sind mit dem dünnen Rohrboden 28 verschweißt, und die gegenüberliegenden Enden der Bündelrohre sind mit dem nicht dargestellten Rohrboden auf der gegenüberliegenden Seite des nicht gezeigten Quenchkühlers 20 verschweißt.

Das Medium des Wasser-/Dampf-Massenstroms strömt gemäß Fig. 4B über die nicht dargestellte sekundäre Zuführrohrleitung 44 zum nicht gezeigten Kühlwassereintritt 25 des sekundären Quenchkühlers 20. Der Massenstrom wird mittels eines an den äußeren Umfang der angeordneten Kühlkanäle 27 angepassten Umlenkbleches 43 zu Eintrittsöffnungen 30 der in Tunnelanordnung ausgebildeten Kühlkanäle 27 geleitet. Der gesamte Massenstrom teilt sich auf die einzelnen Tunnel oder Kühlkanäle 27 auf und passiert ausgehend von den Eintrittsöffnungen 30 unter Umströmung und damit Kühlung der senkrecht zu den Kühlkanälen mit Abstand voneinander durchgesteckten Bündelrohre 29 alle Tunnel oder Kühlkanäle in Richtung von Austrittsöffnungen 31, die mit Abstand fluchtend gegenüber den Eintrittsöffnungen 30 angeordnet sind. Es ergibt sich also eine eindeutig gerichtete Strömung von den Eintrittsöffnungen 30 zu den Austrittsöffnungen 31.

Bei der Durchströmung der Tunnel oder Kühlkanäle 27 dringt ein kleiner Teil des Massenstroms gemäß Fig. 5 durch die einzelnen Ringspalte 19, die jeweils zwischen den Öffnungen 18 der einzelnen Kühlkanale und den senkrecht dazu durchgeführten Bündelrohren 29 ausgebildet sind. Die Ringspalte 19 sind vorzugsweise dafür vorgesehen, dass eine intensive Kühlung der Bündelrohre 29 im Bereich der Ringspalte erfolgen kann, weil ein Teil des Massenstromes durch die Ringspalte 19 tritt und eine wirkungsvolle Wärmeabfuhr erzielt wird.

Hinter den Austrittsöffnungen 31 nach Fig. 4B vereinigen sich die Massenströme wieder und gelangen in einen Mantelraum 36, der das Rohrbündel umschließt und vom Mantel 32 des sekundären Quenchkühlers umgeben ist. Der Mantel 32 ist mit einem Ringflansch 35 verschweißt, der mit dem Rohrboden 28 verbunden ist.

Fig. 4A zeigt einen Schnitt entlang der Linie A - A nach Fig. 3 und Fig. 4B zeigt einen Schnitt entlang der Linie B - B nach Fig. 3.

In Fig. 4A sind die auf dem dünnen Rohrboden 28 durch Stege 33 getrennten und parallel verlaufenden Kühlkanäle 27 oder Tunnel deutlich zu erkennen, die durch das Abdeckblech 34 abgedeckt und durch Stege 33 voneinander getrennt sind, wobei wegen der Klarheit die durch die Öffnungen 18 durchgesteckten Bündelrohre 29 weggelassen sind. Die Kühlkanäle 27 sind auf dem Rohrboden 28 parallel angeordnet, der mit dem Ringflansch 35 verbunden ist, der mit dem Mantel 32 des Quenchkühlers 20 verschweißt ist. Die Kühlkanäle 27 in Tunnelanordnung liegen im Wasser-/Dampfbereich des vom Mantel 32 mit dem eingeschlossenen Rohrbündel umschlossenen Mantelraumes 36. Der Rohrboden 28 mit den darauf angebrachten Kühlkanälen 27 in Tunnelanordnung ist auf der Seite des Gaseintritts 21 oder des Gasaustritts 23 gemäß Pfeilrichtung je nach Schaltung gemäß Fig. 2A oder Fig. 2B des Quenchkühlsystems angeordnet.

In Fig. 4B ist ein Kühlkanal 27 oder Tunnel mit dem Abdeckblech 34 dargestellt, dessen Eintrittsöffnung 30 größer ist als die Austrittsöffnung 31. Der Kühlkanal 27 ist auf dem dünnen Rohrboden 28 angeordnet, der mit dem Ringflansch 35 verbunden ist. Der Ringflansch 35 ist mit dem Mantel 32 des Quenchkühlers 20 verschweißt, der den Mantelraum 36 umgibt. Innerhalb des Mantelraumes 36 ist auf dem Rohrboden 28 unter Bildung einer Wasserkammer 46 das Umlenkblech 43 angeordnet, das an den äußeren Umfang der Kühlkanäle angepasst ist und den Wasser-/Dampf-Massenstrom auf die einzelnen Kühlkanäle 27 aufteilt.

Der in Fig. 4B dargestellte Kühlkanal 27 in Tunnelanordnung weist eine Veränderung des Querschnitts des Tunnels durch eine kontinuierliche Reduzierung der Tunnelhöhe von der Eintrittsöffnung 30 bis zur Austrittsöffnung 31 auf. Die kontinuierliche Reduzierung der Tunnelhöhe zwischen der senkrechten Linie der Austrittsöffnung und dem Abdeckblech ist durch einen Winkel α bestimmt. Der vorbestimmte Winkel ist abhängig von der geforderten Erhöhung der Geschwindigkeit der Strömung über vorbestimmte Bereiche des Rohrbodens und liegt im Bereich von größer/gleich 90° bis 110°.

Fig. 5 zeigt eine Einzelheit X nach Fig.4A, wobei die aus den parallel verlaufenden Stegen 33 und dem Abdeckblech 34 mit den Öffnungen 18 für durchgesteckte Bündelrohre 29 einschließlich der Ringspalte 19 gebildeten Kühlkanäle 27 in Tunnelanordnung in Verbindung mit dem Rohrboden 28 deutlich zu erkennen sind. Umgeben sind die auf dem dünnen Rohrboden 28 angeordneten Kühlkanäle 27 in Tunnelanordnung von dem Ringflansch 35, der mit dem Rohrboden und dem Mantel 32 des Quenchkühlers 20 verbunden ist.

Die Tunnelanordnung wird bei vertikal angeordneten sekundären Quenchkühlern 20 wasser-/dampfseitig immer an den am tiefsten liegenden Stellen des Quenchkühlers angebracht. Dabei ist es nicht wichtig, ob es sich um den Gaseintritt oder den Gasaustritt handelt. Bei horizontal angeordneten sekundären Quenchkühlern 20 ist die Tunnelanordnung wasser-/dampfseitig an der Seite des Gaseintritts 21 angebracht.

Die gesamte Tunnelanordnung der Kühlkanäle 27 oder Tunnel ist von dem Ringflansch 35 umgeben. Eine bevorzugte viereckige Tunnelgeometrie ist im wesentlichen durch drei Bauteile gebildet:
Der dünne Rohrboden 28, der die Gasseite von der Wasser-/Dampfseite trennt und mit dem Ringflansch 35 verbunden ist.

Die Stege 33, welche die einzelnen Wasser-/Dampfströme voneinander trennen, so dass eine eindeutig gerichtete Strömung von den Eintrittsöffnungen 30 in Richtung der Austrittsöffnungen 31 der Kühlkanäle 27 oder Tunnel erzielbar ist, wobei die Stege mit dem Rohrboden 28 verbunden sind.

Das Abdeckblech 34, das eine Begrenzung der Strömung in der Tunnelanordnung der Kühlkanäle 27 sicherstellt und im wesentlichen ein Entweichen der Strömung bis auf den vorgesehenen Anteil, der durch die Ringspalte 19 hindurchtritt, in einen vom Mantel 32 umschlossenen Mantelraum 36 verhindert, der die Bündelrohre 29 des Rohrbündels einschließt. Das Abdeckblech 34 ist mit den Stegen 33 verbunden, insbesondere verschweißt.

Mit der Ausbildung der Kühlkanäle 27 in Tunnelanordnung ist eine eindeutig gerichtete Strömung von den Eintrittsöffnungen 30 in Richtung der Austrittsöffnungen 31 der Kühlkanäle 27 sichergestellt.

In Fig. 6 ist die Darstellung eines Kühlkanals 27 in Tunnelanordnung gemäß Fig. 4B mit dem Flussverlauf des Kühlmediums gezeigt. In der Darstellung ist der Ringflansch 35 gut zu erkennen, der mit dem Rohrboden 28 verbunden ist, auf dem die Kühlkanäle 27 in Tunnelanordnung angebracht sind. Der Ringflansch 35 ist mit dem Mantel 32 des nicht dargestellten Quenchkühlers 20 verbunden, wobei der Mantelraum 36 ausgebildet ist, der die nicht gezeigten Bündelrohre des Rohrbündels umschließt und einen Wasser-/Dampfbereich einschließt.

Das Kühlmedium gelangt gemäß Pfeilrichtung beim Kühlwassereintritt 25 in die Eintrittskammer 46, die sich über eine Hälfte des Umfangs des Mantels 32 erstreckt und im wesentlichen vom Umlenkblech 43 begrenzt ist, das mit dem Rohrboden 28 entlang den Eintrittsöffnungen 30 der Kühlkanäle 27 und dementsprechend mit dem Mantel 32 kurz oberhalb des Kühlwassereintritts verbunden ist, vorzugsweise verschweißt ist. Aus der Eintrittskammer 46 gelangt das Kühlmedium zu den einzelnen Eintrittsöffnungen 30 der Kühlkanäle 27 und verlässt die Kühlkanäle bei den Austrittsöffnungen 31 und gelangt in den Mantelraum 36. Weiterhin ist durch Pfeile angedeutet, dass der Rohrboden 28 auf der Seite des Gaseintritts 21 oder des Gasaustritts 23 je nach Schaltung des Quenchkühlers angeordnet sein kann.

Die vorbestimmte Reduzierung des Querschnitts von der Eintrittsöffnung 30 zur Austrittsöffnung 31 des Kühlkanals 27 oder Tunnels ist für eine Erhöhung der Flussgeschwindigkeit des Wasser-/Dampf-Massenstroms vorgesehen. Die mit der Reduzierung des Querschnitts verbundene Erhöhung der Flussgeschwindigkeit des Massenstroms ist zur intensiveren Kühlung von stark belasteten Teilen des Rohrbodens 28, vor allem der Mitte des Rohrbodens, für eine längere Standzeit des Quenchkühlers 20 und damit des Quenchkühlsystems vorrangig wesentlich.

Die besondere Ausbildung der Kühlkanäle 27 in Tunnelanordnung ist erforderlich, um auszuschließen, dass sich Ablagerungen an der inneren Seite oder Wasserseite des Rohrbodens 28 bilden. Zur Verhinderung von Ablagerungen hat die gerichtete Strömung über den Rohrboden eine definierte Geschwindigkeit aufzuweisen. Daher ist unter Beibehaltung des Massenstroms in den Tunneln die erforderliche Geschwindigkeit unter der Veränderung des Querschnitts der Tunnel anzupassen. Die Veränderung des Querschnitts der Tunnel wird durch eine kontinuierliche Reduzierung der Tunnelhöhe erreicht.

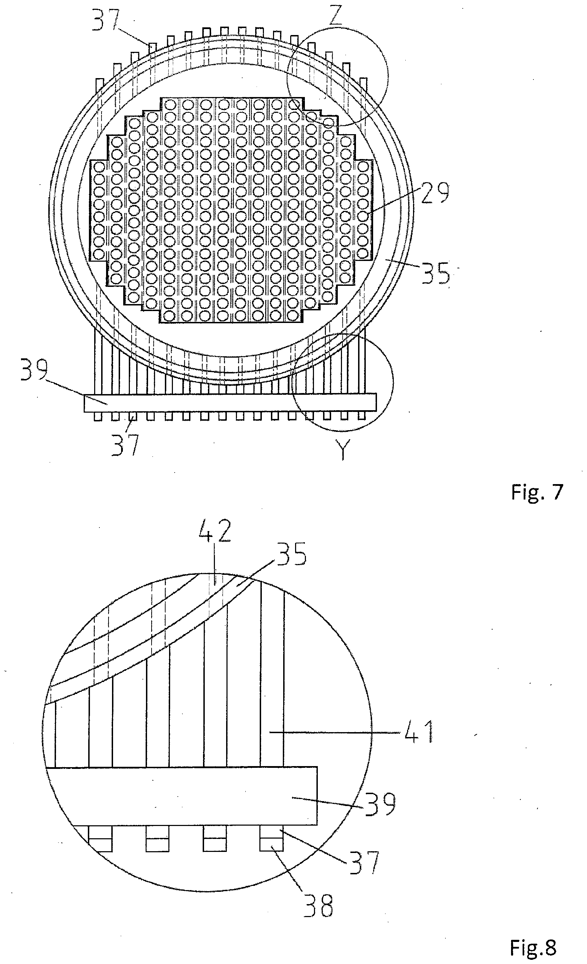

In Fig. 7 ist eine ähnliche Darstellung wie in Fig. 3 gezeigt, wobei jeweils den Eintrittsöffnungen 30 und Austrittsöffnungen 31 der in Tunnelanordnung ausgelegten Kühlkanäle 27 jeweils Inspektions- oder Reinigungsstutzen 37 zugeordnet sind, die fluchtend gegenüberliegend mantelseitig am Ringflansch 35 angebracht sind. Die Inspektions- oder Reinigungsstutzen 37 sind jeweils mit einer Kappe 38 versehen, die im Falle einer wasserseitigen Wartung oder Inspektion der Bündelrohre 29 im Bereich der Tunnelanordnung abtrennbar angebracht sind. Für solche Arbeiten können bei den Inspektions- oder Reinigungsstutzen 37, die sich jeweils gegenüberliegen, die Kappen oder nur einzelne Kappen 38 entfernt werden.

Die abtrennbar angeordneten Kappen 38 der Inspektions- oder Reinigungsstutzen 37 sind als Öffnung oder Zugriff zur Inspektion oder zum Reinigen der Tunnelanordnung der Kühlkanäle 27 vorgesehen. Zur Inspektion oder zum Reinigen werden die Kappen 38 der sich jeweils gegenüberliegenden Inspektions- oder Reinigungsstutzen 37 entfernt. Mittels eines Messgerätes sind bei entfernten Kappen 38 durch die Inspektions- oder Reinigungsstutzen 37 etwaige Ablagerungen erfassbar. Mit Hilfe eines Hochdruckwasserstrahls sind die festgestellten Ablagerungen von einer Öffnung bis zur gegenüberliegenden Öffnung entfernbar. Vorzugsweise werden die mit einem Hochdruckwasserstrahl zu entfernenden Ablagerungen einem Abschlämmsammler 39 zugeführt, der auf einer Seite der Inspektions- oder Reinigungsstutzen 37 angebracht ist und das Abschlämmwasser aufnimmt und ableitet.

In Fig. 8 ist die Einzelheit Y in vergrößertem Maßstab gemäß Fig. 7 gezeigt. Aus Fig. 8 ist deutlich zu erkennen, dass der Abschlämmsammler 39 für die Aufnahme des Abschlämmwassers auf der einen Seite mit Ablaufrohren 41 verbunden ist. Die Ablaufrohre 41 sind am Ringflansch 35 auf Höhe der Tunnelanordnung der nicht gezeigten Kühlkanäle 27 angeschweißt und über Bohrungen 42 im Ringflansch 35 als Zugänge zu der Tunnelanordnung der Kühlkanäle ausgebildet. Die Inspektions- oder Reinigungsstutzen 37 mit den Kappen 38 sind an der anderen Seite des Abschlämmsammlers 39 angeordnet, die den Ablaufrohren 41 gegenüberliegen.

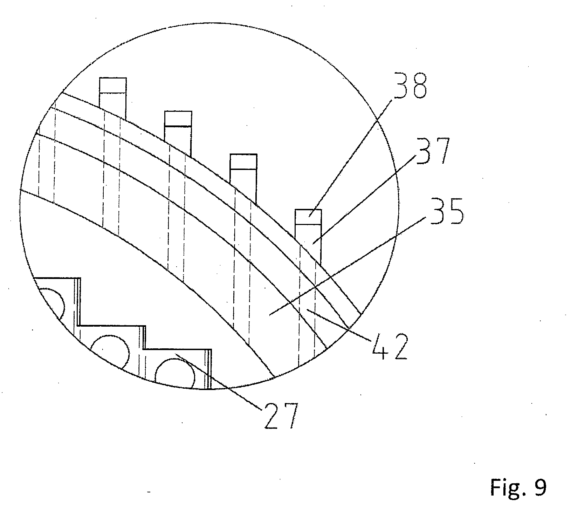

Fig. 9 zeigt eine Einzelheit Z im vergrößerten Maßstab gemäß Fig. 7. Die Inspektions- oder Reinigungsstutzen 37 sind an dem Ringflansch 35 direkt angebracht, und zwar parallel und in einer Linie ausgerichtet zur Richtung der auf der Seite gegenüber der Anordnung des Abschlämmsammlers 39 nach Fig. 8 angeordneten Inspektions- oder Reinigungsstutzen. Über Bohrungen 42 im Ringflansch 35 schaffen die Inspektions- oder Reinigungsstutzen 37 jeweils einen Zugang zu der Tunnelanordnung der Kühlkanäle 27 für eine Inspektion oder Reinigung der Kühlkanäle oder Tunnel.

## Patentansprüche

1. Quenchkühlsystem mit einem primären Quenchkühler (10) als Doppelrohrwärmetauscher und einem Rohrbündel-Wärmetauscher als sekundären Quenchkühler (20) mit mindestens einem Rohrbündel, wobei das Rohrbündel von einem Mantel (32) unter Bildung eines Mantelraumes (36) umschlossen ist, der zwischen zwei mit Abstand voneinander angeordneten Rohrböden (28) ausgebildet ist, zwischen denen Bündelrohre (29) des Rohrbündels jeweils beidseitig in den Rohrböden gehalten sind und wobei der Rohrboden auf der Seite vom Gaseintritt (21) oder Gasaustritt (23) mit den Bündelrohren (29) als dünner Rohrboden ausgebildet ist, wobei der dünne Rohrboden (28) mit parallelen Kühlkanälen (27) versehen ist, die miteinander in Verbindung stehen und von einem Kühlmedium durchströmt sind, **dadurch gekennzeichnet, dass** die Kühlkanäle (27) in Tunnelanordnung ausgebildet und auf dem dünnen Rohrboden (28) als Rohrplatte angeordnet sind, dass die Kühlkanäle (27) in Tunnelanordnung eine viereckige Tunnelgeometrie aufweisen, dass die Kühlkanäle (27) in Tunnelgeometrie gebildet sind (i)aus dem dünnen Rohrboden (28), der eine Gasseite von einer Wasser-/Dampfseite trennt und mit einem Ringflansch (35) verbunden ist, der mit dem Mantel (32) des umschlossenen Rohrbündels verbunden ist; (ii) aus auf dem Rohrboden (28) angeordneten parallelen Stegen (33), die mit dem Rohrboden (28) verbunden sind und einzelne Wasser-/Dampfströme voneinander trennen; (iii) aus einem mit Öffnungen (18) für Bündelrohre (29) versehenem Abdeckblech (34), das mit den Stegen (33) verbunden ist und die Strömung in der Tunnelanordnung der Kühlkanäle (27) begrenzt und ein Entweichen der Strömung in einen vom Mantel (32) des eingeschlossenen Rohrbündels umschlossenen Mantelraum (36) bis auf einen vorbestimmten Anteil verschließt, und dass die in eindeutig gerichtete Strömung von den Eintrittsöffnungen (30) zu den Austrittsöffnungen (31) der Kühlkanäle (27) bewerkstelligen.

2. Quenchkühlsystem nach Anspruch 1, **dadurch gekennzeichnet, dass** mindestens zwei Kühlkanäle (27) in Tunnelanordnung eine Veränderung des Querschnitts des Kühlkanals oder Tunnels durch eine kontinuierliche Reduzierung der Tunnelhöhe von der Eintrittsöffnung (30) bis zur Austrittsöffnung (31) um einen vorbestimmten Winkel α zwischen der senkrechten Linie der Austrittsöffnung und des Abdeckbleches (34) aufweisen.

3. Quenchkühlsystem nach Anspruch 2, **dadurch gekennzeichnet, dass** der vorbestimmte Winkel α abhängig ist von der vorbestimmten Erhöhung der Geschwindigkeit der Strömung des Kühlmediums über vorbestimmte Bereiche des zu kühlenden Rohrbodens (28) und im Bereich von größer/gleich 90° bis 110° liegt.

4. Quenchkühlsystem nach Anspruch 1, **dadurch gekennzeichnet, dass** die Kühlkanäle (27) im Abdeckblech (34) in horizontaler Richtung mit Abstand voneinander die angebrachten Öffnungen (18) aufweisen, dass die Öffnungen (18) unter Bildung von jeweiligen Ringspalten (19) für die jeweils durchgesteckten Bündelrohre (29) ausgelegt sind und dass der jeweilige Ringspalt (19) einen Durchtritt des Kühlmediums zwecks intensiver Kühlung des Bereichs zwischen Bündelrohr (29) und Öffnung (18) bewerkstelligt.

5. Quenchkühlsystem nach Anspruch 1, **dadurch gekennzeichnet, dass** die Kühlkanäle (27) in Tunnelanordnung über Bohrungen (42) im Ringflansch (35) mit Inspektions- oder Reinigungsstutzen (37) kommunizieren, die an der äußeren Oberflächenseite des mit dem Mantel (32) verbundenen Ringflansches (35) jeweils gegenüberliegend und fluchtend mit den Kühlkanälen (27) angebracht sind.

6. Quenchkühlsystem nach Anspruch 4, **dadurch gekennzeichnet, dass** die den Kühlkanälen (27) zugeordneten und jeweils gegenüberliegend und fluchtend mit den Kühlkanälen am Ringflansch (35) angebrachten Inspektions- oder Reinigungsstutzen (37) mit Kappen (38) ausgerüstet sind und dass die Kappen oder einzelne Kappen (38) der jeweils gegenüberliegenden Inspektions- oder Reinigungsstutzen (37) als Öffnung zur wasserseitigen Wartung oder Inspektion der Bündelrohre (29) im Bereich der Kühlkanäle (27) in Tunnelanordnung entfernbar angebracht sind.

7. Quenchkühlsystem nach Anspruch 5, **dadurch gekennzeichnet, dass** die Kappen oder einzelne Kappen (38) der jeweils gegenüberliegend und fluchtend angebrachten Inspektions- oder Reinigungsstutzen (37) als Öffnung zur Reinigung von vorhandenen Ablagerungen im Bereich der Kühlkanäle (27) in Tunnelanordnung entfernbar angebracht sind.

8. Quenchkühlsystem nach Anspruch 4, **dadurch gekennzeichnet, dass** die den Kühlkanälen (27) zugeordneten und jeweils gegenüberliegend und fluchtend am Ringflansch (35) angebrachten Inspektions- oder Reinigungsstutzen (37) über die Bohrungen (42) im Ringflansch (35) und über als Weiterführung der Bohrungen (42) an dem Ringflansch (35) angeschweißte Ablaufrohre (41) mit einem an einer Seite auf Höhe der Kühlkanäle (27) in Tunnelanordnung angebrachten Abschlämmsammler (39) kommunizieren.

9. Quenchkühlsystem nach Anspruch 7, **dadurch gekennzeichnet, dass** die den Kühlkanälen (27) zugeordneten Inspektions- oder Reinigungsstutzen (37) an der den Ablaufrohren (41) gegenüberliegenden Außenseite des Abschlämmsammlers (39) angeordnet sind.

10. Quenchkühlsystem nach Anspruch 8, **dadurch gekennzeichnet, dass** die Inspektions- oder Reinigungsstutzen (37) gegenüber der Seite mit der Anordnung des Abschlämmsammlers (39) direkt am Ringflansch (35) als Weiterführung der Bohrungen (42) im Ringflansch angebracht sind.

11. Quenchkühlsystem nach Anspruch 8, **dadurch gekennzeichnet, dass** über die Inspektions- oder Reinigungsstutzen (37) bei entfernten Kappen (38) ein durchgehender Zugriff auf jeden auf dem Rohrboden (28) angeordneten Kühlkanal (27) erreichbar ist, dass jeder Kühlkanal (27) über ein Einleiten von Wasser als Medium unter Hochdruck in die Inspektions- oder Reinigungsstutzen (37) entweder von beiden Seiten oder von nur einer Seite reinigbar angeordnet ist, und dass der jeweilige Kühlkanal (27) für ein Ablaufen des Abschlämmwassers auf der Ablaufseite über dem zugeordneten Ablaufrohr (41) mit dem angebrachten Abschlämmsammler (39) verbunden ist.

## Claims

1. Quench-cooling system with a primary quench cooler (10) as a double-tube heat exchanger and with a tube bundle heat exchanger as a secondary quench cooler (20) with at least one tube bundle, wherein said tube bundle is enclosed by a casing (32), forming a casing room (36), which is formed between two tube sheets (28) arranged at spaced locations from one another, with bundle tubes (29) of the tube bundle being held between said tube sheets (28) in the tube sheets on both sides, and wherein the tube sheet is designed on the side of the gas inlet (21) or gas outlet (23) with the bundle tubes (29) as a thin tube sheet, wherein the thin tube sheet (28) is provided with parallel cooling channels (27), which are in connection with one another and through which a cooling medium flows, **characterized in that** the cooling channels (27) are designed in a tunnel arrangement and are arranged as a tube plate on the thin tube sheet (28); that the cooling channels (27) in a tunnel arrangement have a rectangular tunnel geometry; that the cooling channels (27) are formed in a tunnel geometry; that the cooling channels (27) are formed with a tunnel geometry (i) from the thin tube sheet (28), which separates a gas side from a water/steam side and is connected to a ring flange (35), which is connected to the casing (32) of the enclosed tube bundle; (ii) from parallel webs (33), which are arranged on the tube sheet (28) and separate individual water/steam flows from one another; (iii) from a covering sheet (34), which is provided with openings (18) for bundle tubes (29) and which is connected to the webs (33) and defines the flow in the tunnel arrangement of the cooling channels (27) and closes the escape of the flow into a casing room (36) enclosed by the casing (32) of the enclosed tube bundle aside from a predetermined percentage; and that the cooling channels (27) designed in a tunnel arrangement bring about an unambiguously directed flow from the inlet holes (30) to the outlet holes (31) of the cooling channels (27).

2. Quench-cooling system in accordance with claim 1, **characterized in that** at least two cooling channels (27) in a tunnel arrangement have a change in the cross section of the cooling channel or tunnel due to a continuous reduction of the tunnel height from the inlet hole (30) to the outlet hole (31) by a predetermined angle α between the vertical line of the outlet hole and of the covering sheet (34).

3. Quench-cooling system in accordance with claim 2, **characterized in that** the predetermined angle α depends on the predetermined increase in the velocity of flow of the cooling medium over predetermined areas of the tube sheet (28) to be cooled and is in the range of greater than/equal to 90° to 110°.

4. Quench-cooling system in accordance with claim 1, **characterized in that** the cooling channels (27) in the covering sheet (34) have the provided openings (18) in the horizontal direction at spaced locations from one another; that the openings (18) are designed such that respective ring clearances (19) are formed for the respective bundle tubes (29) passed through; and that the respective ring clearance (19) brings about the passage of the cooling medium for intensive cooling of the area between the bundle tube (29) and the opening (18).

5. Quench-cooling system in accordance with claim 1, **characterized in that** the cooling channels (27) in a tunnel arrangement communicate via drill holes (42) in the ring flange (35) with inspection or cleaning nozzles (37), which are arranged on the outer surface side of the ring flange (35) connected to the casing (32) opposite each other and flush with the cooling channels (27).

6. Quench-cooling system in accordance with claim 4, **characterized in that** the inspection or cleaning nozzles (37), which are associated with the cooling channels (27) and are arranged opposite and flush with the cooling channels on the ring flange (35), are equipped with covers (38) and that the covers or individual covers (38) of the respective inspection or cleaning nozzles (37) located opposite each other are arranged removably as a hole for the water-side maintenance or inspection of the bundle tubes (29) in the area of the cooling channels (27) in a tunnel arrangement.

7. Quench-cooling system in accordance with claim 5, **characterized in that** the covers or individual covers (38) of the inspection or cleaning nozzles (37) arranged opposite each other and flush are arranged removably as an opening for removing deposits present in the area of the cooling channels (27) in a tunnel arrangement.

8. Quench-cooling system in accordance with claim 4, **characterized in that** the inspection or cleaning nozzles (37), which are associated with the cooling channels (27) and are arranged opposite each other and flush with one another on the ring flange (35), communicate with a boiler blow-down tank (39) arranged on one side at the level of the cooling channels (27) in a tunnel arrangement via the drill holes (42) in the ring flange (35) and via welded-on drain pipes (41) as an extension of the drill holes (42) on the ring flange (35).

9. Quench-cooling system in accordance with claim 7 **characterized in that** the inspection or cleaning nozzles (37) associated with the cooling channels (27) are arranged on the outer side of the boiler blow-down tank (39), which side is located opposite the drain pipes (41).

10. Quench-cooling system in accordance with claim 8, **characterized in that** the inspection or cleaning nozzles (37) are arranged directly on the ring flange (35) opposite the side on which the boiler blow-down tank (39) is arranged as a continuation of the drill holes (42) in the ring flange.

11. Quench-cooling system in accordance with claim 8, **characterized in that** with the covers (38) removed, a continuous access can be obtained to each cooling channel (27) arranged on the tube sheet (28) via the inspection or cleaning nozzles (37); that each cooling channel (27) is arranged such that it can be cleaned either from both sides or from only one side by introducing water as a medium under high pressure into the inspection or cleaning nozzles (37); and that the respective cooling channel (27) is connected to the provided boiler blow-down tank (39) for draining the blow-down water on the drain side via the associated drain pipe (41).

## Revendications

1. Système de refroidissement à trempe, avec un refroidisseur à trempe (10) primaire en guise d'échangeur thermique à double tube et un échangeur thermique à faisceau tubulaire en guise de refroidisseur à trempe (20) secondaire, avec au moins un faisceau tubulaire, le faisceau tubulaire étant entouré d'une enveloppe (32) en formant un espace d'enveloppe (36) qui est conçu entre deux fonds de tube (28) placés avec un écart mutuel entre lesquels des tubes (29) du faisceau tubulaire sont maintenus chacun de part et d'autre dans les fonds de tube et le fond de tube étant conçu du côté de l'entrée de gaz (21) ou de la sortie du gaz (23) avec les tubes (29) en tant que fond de tube mince, le fond de tube (28) mince étant muni de canaux de refroidissement (27) parallèles, qui sont assemblés les uns aux autres et qui sont traversés par un fluide de refroidissement, **caractérisé en ce que** les canaux de refroidissement (27) sont agencés en tunnel et sont placés sur le fond de tube (28) mince en tant que plaque tubulaire, **en ce que** les canaux de refroidissement (27) agencés en tunnel présentent une géométrie de tunnel rectangulaire, **en ce que** les canaux de refroidissement (27) sont formés avec une géométrie en tunnel (i) à partir du fond de tube (28) mince, qui sépare un côté gaz d'un côté eau/vapeur et qui est assemblé avec une bride annulaire (35) qui est assemblée avec l'enveloppe (32) du faisceau tubulaires entouré ; (ii) à partir de barrettes parallèles (33) placées sur le fond de tube (28), qui sont assemblées avec le fond de tube (28) et séparent les uns des autres des flux individuels d'eau/de vapeur ; (iii) à partir d'une tôle de recouvrement (34) munie d'orifices (18) pour des tubes (29) qui est assemblée avec les barrettes (33) et qui limite l'écoulement dans l'agencement en tunnel des canaux de refroidissement (27) et qui obture un échappement de l'écoulement dans un espace d'enveloppe (36) entouré par l'enveloppe (32) du faisceau tubulaire enfermé jusqu'à une fraction prédéfinie et **en ce que** les canaux de refroidissement (27) agencés en tunnel provoquent un écoulement clairement orienté des orifices d'entrée (30) vers les orifices de sortie (31) des canaux de refroidissement (27).

2. Système de refroidissement à trempe selon la revendication 1, **caractérisé en ce qu'**au moins deux canaux de refroidissement (27) dans l'agencement en tunnel présentent une modification de la section transversale du canal de refroidissement ou du tunnel par une réduction continue de la hauteur du tunnel, de l'orifice d'entrée (30) jusqu'à l'orifice de sortie (31) d'un angle prédéfini α entre la ligne verticale de l'orifice de sortie et la tôle de recouvrement (34).

3. Système de refroidissement à trempe selon la revendication 2, **caractérisé en ce que** l'angle prédéfini α dépend de l'augmentation prédéfinie de la vitesse de l'écoulement du fluide de refroidissement sur des zones prédéfinies du fond de tube (28) à refroidir et se situe dans l'ordre supérieur/égal à de 90° à 110°.

4. Système de refroidissement à trempe selon la revendication 1, **caractérisé en ce que** les canaux de refroidissement (27) dans la tôle de recouvrement (34) comportent dans la direction horizontale avec un écart mutuel les orifices (18) ménagés, **en ce qu'**en formant des interstices annulaires (19) respectifs, les orifices (18) sont dimensionnés pour les tubes (29) respectivement enfilés, **en ce que** l'interstice annulaire (19) respectif provoque un passage du fluide de refroidissement pour assurer un refroidissement intensif de la zone entre le tube (29) et l'orifice (18).

5. Système de refroidissement à trempe selon la revendication 1, **caractérisé en ce que** les canaux de refroidissement (27) agencés en tunnel communiquent par l'intermédiaire de perçages (42) dans la bride annulaire (35) avec des tubulures d'inspection ou de nettoyage (37) qui sont montées sur le côté superficiel extérieur de la bride annulaire (35) assemblée avec l'enveloppe (32), chaque fois à l'opposée l'une de l'autre et en alignement sur les canaux de refroidissement (27).

6. Système de refroidissement à trempe selon la revendication 4, **caractérisé en ce que** les tubulures d'inspection ou de nettoyage (37) associées aux canaux de refroidissement (27) et montées sur la bride annulaire (35), chaque fois à l'opposée l'une de l'autre et en alignement sur les canaux de refroidissement sont équipées de calottes (38) et **en ce que** les calottes ou des calottes (38) individuelles des tubulures d'inspection ou de nettoyage (37) chaque fois opposées sont montées de manière amovible, en tant qu'orifice pour la maintenance côté eau ou pour l'inspection des tubes (29) dans la zone des canaux de refroidissement (27) agencés en tunnel.

7. Système de refroidissement à trempe selon la revendication 5, **caractérisé en ce que** les calottes ou des calottes (38) individuelles des tubulures d'inspection ou de nettoyage (37) chaque fois montées à l'opposée et en alignement sont montées de manière amovible en tant qu'orifice pour le nettoyage de dépôts présents dans la zone des canaux de refroidissement (27) agencés en tunnel.

8. Système de refroidissement à trempe selon la revendication 4, **caractérisé en ce que** les tubulures d'inspection ou de nettoyage (37) associées aux canaux de refroidissement (27) et montées sur la bride annulaire (35), chaque fois à l'opposée l'une de l'autre et en alignement sur les canaux de refroidissement communiquent par l'intermédiaire des perçages (42) dans la bride annulaire (35) et par l'intermédiaire de tubes d'évacuation (41) soudés sur la bride annulaire (35) en guise de prolongement des perçages (42) avec un collecteur de débourbage (39) monté sur un côté, au niveau des canaux de refroidissement (27) agencés en tunnel.

9. Système de refroidissement à trempe selon la revendication 7, **caractérisé en ce que** les tubulures d'inspection ou de nettoyage (37) associées aux canaux de refroidissement (27) sont placées sur le côté extérieur opposé aux tubes d'écoulement du collecteur de débourbage (39).

10. Système de refroidissement à trempe selon la revendication 8, **caractérisé en ce que** les tubulures d'inspection ou de nettoyage (37) sont montées à l'opposée de l'agencement du collecteur de débourbage (39), directement sur la bride annulaire (35) en tant que prolongement der perçages (42) dans la bride annulaire.

11. Système de refroidissement à trempe selon la revendication 8, **caractérisé en ce que**, lorsque les calottes (38) sont retirées, par l'intermédiaire des tubulures d'inspection ou de nettoyage (37), un accès traversant à chaque canal de refroidissement (27) placé sur le fond de tube (28) peut être obtenu, **en ce que** chaque canal de refroidissement (27) est placé de manière à pouvoir être nettoyé par une introduction d'eau en tant que fluide sous haute pression dans les tubulures d'inspection ou de nettoyage (37) soit par les deux côtés ou par un seul côté, et **en ce que** pour une évacuation de l'eau de débourbage, le canal de refroidissement (27) respectif est assemblé sur le côté évacuation par l'intermédiaire du tube d'évacuation (41) associé avec le collecteur de débourbage (39) monté.
